Europäisches Patentamt

European Patent Office

Office européen des brevets

(19)

(11) EP 0 729 744 A1

(12) DEMANDE DE BREVET EUROPEEN

(43) Date de publication:
04.09.1996 Bulletin 1996/36

(51) Int Cl.⁶: A61K 7/42

(21) Numéro de dépôt: 96400418.8

(22) Date de dépôt: 28.02.1996

(84) Etats contractants désignés:
AT BE CH DE DK ES FR GB GR IE IT LI LU NL PT SE

(30) Priorité: 03.03.1995 FR 9502481

(71) Demandeur: ROUSSEL-UCLAF
93230 Romainville (FR)

(72) Inventeurs:
• Bobier-Rival, Carinne
F-95000 Cergy (FR)
• Fournier, Odile
F-92500 Rueil Malmaison (FR)
• Fructus, Alain
F-92400 Courbevoie (FR)

(74) Mandataire: Vieillefosse, Jean-Claude et al
ROUSSEL UCLAF,
Département des Brevets,
111, Route de Noisy
93235 Romainville Cédex (FR)

(54) Composition cosmétiques pour la protection solaire

(57) L'invention a pour objet des compositions cosmétiques pour la protection solaire contenant un ou plusieurs filtres solaires et un mélange de deux ou plusieurs solvants pour lesdits filtres solaires.

## Description

L'invention concerne des compositions cosmétiques pour la protection solaire et le procédé de préparation de ces compositions.

La protection de la peau contre les coups de soleil et autres lésions associées avec l'exposition aux radiations ultra-violet, a obtenu une grande importance, puisqu'un nombre de personnes plus grand que jamais s'adonne aux activités de loisirs à l'extérieur en s'exposant de plus en plus à la lumière du soleil.

Depuis longtemps l'industrie cosmétique met à la disposition des personnes, des produits tels que des gels, des crèmes, des huiles contenant des filtres solaires et des réflecteurs solaires. Ce sont des substances chimiques, organiques ou inorganiques qui absorbent ou reflètent les rayonnements ultra violets UVB et/ou UVA.

Il est en effet intéressant de limiter les effets du rayonnement solaire au niveau cutané, en particulier ceux des UVB et UVA. Les premiers provoquent principalement l'érythème solaire et à plus long terme des altérations cutanées bénignes et malignes, les seconds sont le principal facteur du vieillissement cutané. D'une façon générale l'excès des rayons UV est néfaste pour la peau.

Les produits protecteurs solaires sont d'une très grande utilité car ils apportent réellement une protection de la peau qui est mesurée par ce que l'on appelle un facteur de protection.

Cependant, il convient d'apporter le maximum de protection avec le taux minimum de filtres solaires, c'est-à-dire qu'il faut optimiser le rapport facteur de protection sur taux de filtres.

Il apparaissait donc judicieux de trouver de nouvelles compositions cosmétiques qui limitent les effets du rayonnement sans être agressives pour la peau.

Dans l'art antérieur, notamment dans le brevet FR-B 2615860 et dans la demande de brevet EP-A 509904, sont décrites des dispersions huileuses et crèmes solaires mais ces compositions ne présentent pas de facteurs de protection satisfaisants.

C'est l'objet de la présente invention d'utiliser des compositions chimiques qui synergisent l'action des substances filtrantes UV.

Parmi les filtres organiques connus figurent : l'octyl méthoxycinnamate (limité à 10 % en poids maximum) filtre UVB, le butyl méthoxydibenzoylméthane (limité à 5 % en poids) filtre UVA, et le 4-méthylbenzylidène camphor, filtre UVB (limité à 6 %). Or le butyl méthoxydibenzoylméthane se présente à l'état cristallisé et il est très peu soluble dans les substances huileuses et solvants habituellement utilisées en cosmétologie. Un des meilleurs solubilisants du butyl méthoxydibenzoylméthane est l'octyl méthoxycinnamate. Toutefois, l'usage de l'octyl méthoxycinnamate étant limité à une teneur de 10 % en poids maximum dans un produit solaire et la solubilité du butyl méthoxydibenzoylméthane dans l'octyl méthoxycinnamate étant de 20 % en poids du premier pour 80 % en poids du second, il est évident qu'on est ainsi limité à environ 2 % en poids maximum de butyl méthoxydibenzoylméthane dans une formule de produit solaire. Il a été montré cependant que l'apport de filtre UVA dans une formule augmente très sensiblement son pouvoir de protection vis-à-vis de l'érythème solaire, et permet d'éviter des dégradations de l'épiderme et du derme conduisant au vieillissement de la peau.

Il était donc intéressant d'essayer de dépasser ce taux de butyl méthoxydibenzoylméthane (Parsol 1789) dans un produit solaire.

Selon la présente invention, on a trouvé que parmi les quelques rares solubilisants des filtres solaires du type butyl méthoxydibenzoylméthane l'association de deux solvants, non seulement favorise la dissolution du filtre solaire du type Parsol 1789, mais de plus synergise l'absorption des rayons UV et augmente le facteur de protection du produit solaire.

L'invention concerne donc des compositions cosmétiques pour la protection solaire contenant un ou plusieurs filtres solaires et un mélange de deux ou plusieurs solvants pour lesdits filtres solaires.

Les deux solvants sont préférentiellement le néopentanoate d'isostéaryl, utilisé par exemple dans la proportion de 0,5 à 10 % (en poids), préférentiellement de 1 à 5 %, et le polyéthylène glycol (comme par exemple le PEG-400), utilisé par exemple dans la proportion de 1 à 12 % (en poids), préférentiellement de 2 à 8 %.

D'autres filtres solaires comme le 4-méthylbenzylidène camphor sont également peu solubles, et l'utilisation de ce mélange de deux solvants limite les risques de recristallisation dans la composition et augmente son pouvoir filtrant.

L'invention concerne particulièrement des compositions cosmétiques pour la protection solaire contenant comme filtres solaires, de l'octylméthoxycinnamate et du butylméthoxydibenzoylméthane.

La composition contient de préférence le butylméthoxydibenzoylméthane en proportion de 20 à 50 % en poids par rapport à l'octylméthoxycinnamate. La composition est préférentiellement une émulsion du type eau dans huile ou eau dans huile de silicone. On préfère utiliser la combinaison de trois à quatre filtres solaires différents.

La composition cosmétique peut contenir 0,001 à 4 % du poids total de la composition d'un composé à activité anti-inflammatoire choisi par exemple parmi les biolysats Hafnia, acide 18β glycyrrhétinique ou enoxolone et ses dérivés, glycyrrhétinate d'ammonium, stéaryl glycyrrhétinate, et son complexe avec les phospholipides (phytosomes (R)), l'α bisabolol, l'alcool batylique, l'extrait d'hirudine, l'extrait de meristem, l'extrait d'ougon, l'extrait de mimosa tenuiflora, l'extrait de karité, le panthénol, l'acide ximménique, l'huile de maïs peroxydée.

Notamment, l'ajout d'un tel composé peut diminuer l'état inflammatoire de la peau.

Parmi ces composés à activité anti-inflammatoire, on citera de façon préférentielle, les biolysats Hafnia, et notamment dans la proportion de 0,001 à 1 % de biolysat Hafnia, dans les compositions telles que définies ci-dessus.

Le brevet FR-B 84 06559 décrit notamment de tels biolysats Hafnia.

Les compositions cosmétiques selon l'invention peuvent contenir aussi d'autres additifs, comme d'autres huiles, des parfums, des agents dispersants, des émulsifiants, d'autres filtres solaires ou des conservateurs.

L'huile pour la composition cosmétique peut être n'importe quelle huile dont la présence est souhaitable dans la composition résultante. Ces huiles sont ordinairement les huiles végétales comme l'huile de jojoba, d'olive, d'avocat, de macadamia, des huiles minérales comme l'huile de vaseline, des hydrocarbures comme le dioctyl cyclohexane ou l'isohexadécane, des esters comme le palmitate de cétyle ou l'isocétyle, des cires naturelles comme la cire d'abeille, de carnauba, de candellila, des triglycérides semi synthétiques comme les triglycérides capryliques ou capriques, des alcools gras comme l'alcool cétylique ou stéarylique, des esters du propylène glycol comme le monostéarate de propylène glycol, des esters de l'acide benzoïque comme le FINSOL TN® (Witco USA), les diesters d'acides gras saturés, par exemple le 12-stéaroyl-stéarate de 2-octyl-dodécyle, l'alcool oléylique et le palmitate d'isopropyle, le tétracaprylate/ caprate de pentaérythritol, les diesters de propylèneglycol d'acides gras de noix de coco et le tétraisostéarate de pentaérythritol.

Les compositions peuvent contenir un agent dispersant organique.

Comme conservateurs on peut utiliser par exemple le méthylparaben, le propylparaben, le O-cymen-5-ol ou le 2-bromo 2-nitropropane 1,3-diol.

L'invention concerne aussi un procédé de préparation d'une composition cosmétique telle que définie ci-dessus, caractérisé en ce que l'on prépare une émulsion avec

a) une phase grasse comprenant le premier solvant et un ou plusieurs filtres solaires et

b) avec une phase aqueuse comprenant le deuxième solvant et éventuellement un autre filtre solaire.

De préférence les deux phases sont chauffées à une température de 40° à 70°C puis mélangées vigoureusement pendant plusieurs minutes.

Le procédé de préparation dépend du type de composition.

Il est aussi possible d'ajouter le ou les filtres solaires à l'émulsion.

La présente invention a aussi pour objet une méthode de traitement cosmétique caractérisée en ce que l'on utilise les compositions cosmétiques telles que définies ci-dessus. Dans la méthode de traitement cosmétique utilisant les compositions de l'invention, lesdites compositions sont utilisées par application cutanée comme les compositions usuelles.

La présente invention a aussi pour objet à titre de médicament cosmétique, des compositions telles que définies ci-dessus.

## EXEMPLE 1 :

Pour montrer l'effet de l'association des deux solvants, on prépare les trois émulsions solaires suivantes : Emulsion I contenant 2 % de néopentanoate d'isostéaryl et 4,5 % de PEG-400,
Emulsion II contenant 2 % de néopentanoate d'isostéaryl et 0 % de PEG-400,
Emulsion III contenant 4,5 % de PEG-400 et 0 % de néopentanoate d'isostéaryl.

On compare leur pouvoir filtrant par la méthode ex-vivo dérivant de la méthode de DIFFEY et ROBSON (J. Soc. Cosmet. Chem. 40, 127-132, 1989).

Le pouvoir filtrant des trois compositions est assuré par la combinaison des filtres solaires suivants :

- octyl méthoxycinnamate 10 %
- butyl méthoxydibenzoylméthane 5 %
- 4-méthylbenzylidène camphor 3 %
- acide phénylbenzimidazole sulfonique 1,5 %.

L'émulsion est du type eau dans silicone et comprend les éléments suivants :

**Phase grasse :**

|  | Emulsion | | |
|---|---|---|---|
|  | I en g | II en g | III en g |
| Cétyl Diméthicone copolyol | 4 | 4 | 4 |
| Céramides HO$_3$® (Sederma) | 0,1 | 0,1 | 0,1 |
| Huile de jojoba | 1 | 1 | 1 |
| PEG-45 Dodécyl glycol copolymer (Akzo ; Elfacos® ST9) | 1 | 1 | 1 |
| Ozokerite (paraffine minérale) | 0,6 | 0,6 | 0,6 |
| Alkyl benzoate | 3,5 | 3,5 | 3,5 |
| Octyl méthoxycinnamate | 10 | 10 | 10 |
| Butyl méthoxydibenzoylméthane | 5 | 5 | 5 |
| 4-méthylbenzylidène camphor | 3 | 3 | 3 |
| Huile de silicone | 2 | 2 | 2 |
| Tocophérol acétate | 0,15 | 0,15 | 0,15 |
| Néopentanoate d'isostéaryl | 2 | 2 | - |

## Phase aqueuse :

| | Emulsion | | |
| --- | --- | --- | --- |
| | I<br>en g | II<br>en g | III<br>en g |
| Eau    Q.S. | 100 | 100 | 100 |
| Glycérine | 3 | 3 | 3 |
| Sclerosium gomme | 0,2 | 0,2 | 0,2 |
| EDTA | 0,1 | 0,1 | 0,1 |
| Chlorure de sodium | 0,8 | 0,8 | 0,8 |
| Acide phénylbenzimidazole-sulfonique | 1,5 | 1,5 | 1,5 |
| Soude caustique pure | 0,2 | 0,2 | 0,2 |
| PEG-400 | 4,5 | - | 4,5 |
| Conservateurs (parabens, biosol) | 0,5 | 0,5 | 0,5 |

Les deux phases sont chauffées à 60°C, puis mélangées vigoureusement pendant 5 minutes. Quand l'émulsion est bien formée, on refroidit jusqu'à température ambiante sous agitation douce.

**EXEMPLE 2** :

Test d'évaluation de l'indice de protection ex-vivo de trois produits de l'exemple 1.

L'évaluation du pouvoir photoprotecteur des produits solaires chez l'homme contre les UVB repose sur la détermination de la dose érythémale minimum (DEM).

Actuellement il existe plusieurs protocoles d'évaluation du pouvoir photoprotecteur des produits solaires contre les radiations ultraviolettes B (UVB) chez l'homme (FDA, DIN, Australian Standards etc.). Les conditions opératoires varient d'un protocole à un autre, fait qui conduit à des valeurs d'indices de protection solaires (IP) différents pour la même formule.

Plusieurs méthodes in vitro ont été développées au cours du temps, ayant comme but d'éviter l'expérimentation chez l'animal et de réduire au maximum l'évaluation chez l'homme qui est coûteuse et nécessite de longs délais.

Si toutefois l'évaluation chez l'homme reste nécessaire pour déterminer le pouvoir photoprotecteur d'un produit fini, les techniques in vitro ou ex-vivo représentent un moyen rapide d'évaluation au cours du développement galénique des produits solaires.

Parmi les différentes techniques employées, les méthodes spectrophotométriques semblent les plus appropriées pour ce type d'expérimentation. Le principe de toutes ces méthodes consiste à déterminer le spectre d'absorption ou de transmission des rayons ultraviolets de différents supports en absence, puis en présence d'un produit solaire à étudier.

Récemment, une nouvelle méthode mise au point par Diffey et Robson utilise un support synthétique, un Spectroradiomètre comme appareil de mesure et une lampe au Xénon comme source UV (Int. J. Cosm. Sci. 11, 245-249, 1989).

Si ce support synthétique donne un spectre d'absorption des UV entre 290 nm et 400 nm similaire à l'épiderme humain, il n'est pas approprié à l'étude des préparations à base d'alcool, et aux préparations à phase externe huileuse.

Une autre équipe de chercheurs a proposé comme support du stratum corneum (2 à 4 couches fixées à l'aide d'une colle à base de cyanoacrylate, sur une plaque en quartz) (Int. J. Cosm. Sci. 15, 234-244, 1993).

Les résultats obtenus montrent une corrélation linéaire sous-estimée par rapport à ceux obtenus chez l'homme d'après la norme australienne (Australian Standards).

La présence du cyanoacrylate pourrait induire un comportement différent du produit appliqué et l'épaisseur réduite du stratum corneum (2 à 4 couches), pourrait modifier les phénomènes d'imprégnation.

Dans cette demande, une méthode utilisant comme support l'épiderme humain a été appliquée. Des mesures spectrophotométriques ont été prises en transmission diffuse. La sensibilité du système a été augmentée par l'utilisation d'une sphère d'intégration.

Afin de pouvoir corréler les mesures purement physiques obtenues par spectrophotométrie aux indices de protection obtenus in vivo chez l'homme, plusieurs formules de calcul ont été proposées.

La méthode utilisée dans cette demande de brevet est une variante du calcul proposé par Diffey et Robson.

**Matériel et méthodes**

Un spectrophotomètre (Cary III VARIAN UV-VIS) et une lampe UV (Deutérium) d'une puissance de 27 watts ont été utilisés. Le taux de lumière parasite dans le domaine UV est inférieur à 1 %. L'appareil est équipé d'une Sphère d'Intégration UV-VIS (NIR DMS ACCESSORIES). Le revêtement intérieur de la sphère est constitué de polyfluoréthylène qui a un indice de réflexion de 0,95 dans le domaine UV. De plus la présence du détecteur au milieu de la sphère permet d'augmenter la sensibilité du système. Cela est particulièrement intéressant dans le contexte de notre expérimentation car la totalité du rayonnement émergeant atteint le photomultiplicateur.

**Conditions opératoires**

Le support utilisé pour l'application des produits est l'épiderme humain. La peau humaine est prélevée au niveau abdominal chez des sujets caucasiens sains, lors d'intervention de chirurgie esthétique. La séparation de l'épiderme du derme est réalisée selon la technique de Christophers et Kligman (Arch. Dermatol. 88, 701-705, 1963).

L'épiderme est ensuite étalé sur une lame en quartz et laissé à l'air libre pendant 7 minutes avant le début de la mesure.

Dans l'expérimentation des conditions opératoires préconisées par la méthode d'évaluation chez l'homme d'après la norme FDA sont choisis :

a) la quantité de produit déposée à la surface du support est de 2 mg/cm$^3$ ;
b) l'étalement du produit est réalisé uniformément sur toute la surface à l'aide d'un doigtier en caoutchouc ;
c) la prise de mesure est faite après un repos d'environ 10 minutes à l'air libre de l'ensemble constitué par la plaque en quartz/l'épiderme/le produit. Ce temps de repos est nécessaire à l'imprégnation du stratum corneum par le produit et à l'élimination de l'excès d'humidité.

Le principe de la méthode consiste à déterminer le spectre de transmission diffuse des rayons UV (290-400 nm) à travers l'épiderme avant et après application du produit solaire à étudier. Chaque préparation a été testée sur des échantillons d'épiderme provenant de différents sujets (3 pour chaque produit). Sur chaque morceau d'épiderme 2 mesures ont été effectuées à deux endroits différents. Le pourcentage de transmission avant et après application du produit a été calculé sur la moyenne de 6 mesures pour chaque produit solaire. Une étude préliminaire concernant la reproductibilité des mesures avant et après application du produit a été réalisée.

**Expression des résultats**

Les valeurs du pourcentage de transmission % T(l) entre 290 nm et 400 nm avec un pas de 5 nm (soit 23 valeurs) obtenues avant et après application du produit ont servi au calcul des facteurs de protection monochromatiques (FPM).

$$FPM\ (I) = \%\ T(I)\ \text{"épiderme"} / \%\ T\ (I)\ \text{"épiderme + produit"}.$$

Des facteurs de protection FP prévisionnels ont été calculés d'après la formule proposée par Diffey et Robson :

$$FP = \int_{290}^{400} E(I) * e(II) / \int_{290}^{400} E(I) * e(I)/FPM$$

E(l) = intensité d'irradiation spectrale de la lumière solaire
e(l) = spectre de référence pour l'action érythémale.

L'intensité d'irradiation spectrale de la lumière solaire a été mesurée dans des conditions définies : à midi, à une latitude de 40°N, un angle solaire de 20° par rapport au zénith et une épaisseur de la couche d'ozone de 0,305 cm.

Le spectre d'action érythémale rassemble des valeurs chiffrées établies en fonction de l'action érythémale induite par un rayonnement de longueur d'onde donné (I). Il a été adopté par la Commission Internationale de l'Eclairage (CIE) comme spectre d'action érythémale de référence. Ainsi un facteur de protection global a été calculé pour chaque formulation.

Le pourcentage de transmission des radiations UV à travers l'épiderme varie selon le phototype du sujet, plus exactement selon la teneur en mélanines de l'épiderme.

**Résultats obtenus pour les 3 émulsions I, II et III**.

|  | Emulsion I | Emulsion II | Emulsion III |
|---|---|---|---|
| Facteur de protection | 112 | 63,1 | 77,2 |

On voit donc que la composition contenant les deux solvants a un facteur de protection solaire nettement supérieur à ceux des deux compositions ne contenant chacune qu'un seul solvant pour les filtres solaires.

Ce type de composition permet donc, pour une quantité donnée de filtres solaires, d'avoir un facteur de protection supérieur, ou inversement pour un facteur de protection souhaité, permet d'inclure moins de filtres solaires dans la composition, limitant par là les effets indésirables.

**Revendications**

1. Compositions cosmétiques pour la protection solaire, contenant un ou plusieurs filtres solaires et un mélange de deux ou plusieurs solvants pour lesdits filtres solaires.

2. Compositions cosmétiques selon la revendication 1, caractérisées par le fait que les deux solvants sont le polyéthylène-glycol et le néopentanoate d'isostéaryl.

3. Compositions cosmétiques selon les revendications 1 et 2, caractérisées par le fait qu'elles contiennent de 0,5 à 10 % en poids de néopentanoate d'isostéaryl et de 1 à 12 % en poids de polyéthylène-glycol.

4. Compositions cosmétiques selon les revendications 1 à 3, caractérisées par le fait qu'elles contiennent de 1 à 5 % en poids de néopentanoate d'isostéaryl et de 2 à 8 % en poids de polyéthylène-glycol 400.

5. Compositions cosmétiques selon les revendications 1 à 4, caractérisées par le fait qu'elles contiennent comme filtres solaires de l'octylméthoxycinnamate et du butylméthoxydibenzoylméthane.

6. Compositions cosmétiques selon les revendications 1 à 5, caractérisées par le fait que le butylméthoxydibenzoylméthane est en proportion de 20 à 50 % en poids par rapport à l'octylméthoxycinnamate.

7. Compositions cosmétiques selon les revendications 1 à 6, caractérisées par le fait que l'on utilise des émulsions du type eau dans huile.

8. Procédé de préparation d'une composition cosmétique contenant un ou plusieurs filtres solaires et un mélange de deux ou plusieurs solvants pour lesdits filtres solaires, caractérisé en ce que l'on prépare une émulsion avec une phase grasse comprenant le premier solvant et un ou plusieurs filtres solaires et avec une phase aqueuse comprenant le deuxième solvant et éventuellement un autre filtre solaire.

9. Méthode de traitement cosmétique caractérisée en ce que l'on utilise les compositions cosmétiques selon les revendications 1 à 7.

EP 0 729 744 A1

Office européen
des brevets

**RAPPORT DE RECHERCHE EUROPEENNE**

Numero de la demande
EP 96 40 0418

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | EP-A-0 430 473 (RICHARDSON-VICKS INC.) 5 Juin 1991 <br> * le document en entier * <br> --- | 1-9 | A61K7/42 |
| A | US-A-5 106 838 (REINHART) 21 Avril 1992 <br> * le document en entier * <br> --- | 1-9 | |
| A | US-A-4 810 489 (MURRAY ET AL.) 7 Mars 1989 <br> * le document en entier * <br> --- | 1-9 | |
| A | US-A-4 917 882 (STROBRIDGE) 17 Avril 1990 <br> * le document en entier * <br> --- | 1-9 | |
| A | WO-A-92 17159 (RICHARDSON-VICKS INC.) 15 Octobre 1992 <br> * le document en entier * <br> --- | 1-9 | |
| A | US-A-5 008 100 (ZECCHINO ET AL.) 16 Avril 1991 <br> * le document en entier * <br> --- | 1-9 | **DOMAINES TECHNIQUES RECHERCHES (Int.Cl.6)** |
| A | EP-A-0 427 411 (RICHARDSON-VICKS INC.) 15 Mai 1991 <br> * le document en entier * <br> ----- | 1-9 | A61K |

Le présent rapport a été établi pour toutes les revendications

| Lieu de la recherche | Date d'achèvement de la recherche | Examinateur |
|---|---|---|
| LA HAYE | 19 Juin 1996 | Couckuyt, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)